# EUROPEAN PATENT APPLICATION

(11) **EP 2 801 343 A1**
(43) Date of publication of application: **12.11.2014**
(21) Application number: 14165765.0
(22) Date of filing: 24.04.2014
(51) Int. Cl.: A61F 9/00, A61F 9/013

(54) **Preoperative toric axis corneal marker**

(30) Priority: 07.05.2013 ES 201330656
(71) Applicant: Fernandez Cambra, Francisco Javier, 50003 Zaragoza (ES)
(72) Inventor: Fernandez Cambra, Francisco Javier, 50003 Zaragoza (ES)
(74) Representative: Ungria López, Javier

(57) **Abstract**

Preoperative toric axis corneal marker useful for delimiting the correct position of an intraocular lens, implanting an intraestromal ring in a patient's eye, performing a limbal relaxing incision or any other type of intervention requiring a precise marking prior to an intervention on the cornea, wherein the marker incorporates an attaching means to a slit lamp and which marker comprises a generally "L"-shaped body which is anchored, by means of a first wing, to the attaching means to the slit lamp and its second wing features a hollow cylindrical shape where a tubular bushing, which presents at least a small lug radially arranged at its free base, is fitted, through which the marking on the eye will be accomplished.

## Description

### OBJECT OF THE INVENTION

As the title indicates, this invention refers to a preoperative toric axis corneal marker, with the purpose of determining the correct position of an intraocular lens, implanting an intrastromal ring in a patient's eye, performing a limbal relaxing incision or any other type of intervention requiring a precise marking prior to a surgery on the patient's eye.

The preoperative toric axis corneal marker object of the invention is associated to a conventional and known slit lamp, which is used for the eye observation.

### TECHNICAL FIELD

A preoperative toric axis corneal marker is described herein, which is applicable for accurate marking of the toric axis of an intraocular lens, implanting an intrastromal ring, performing relaxing sclero-limbal incisions or any other type of surgery requiring prior and precise marking on the cornea by the surgeon, said marking being performed before the patient enters to the operating room for the proper performance of the later technique.

### BACKGROUND OF THE INVENTION

As it is well known, for the observation and examination of the eyes, slit lamps, based on a magnifying microscope, are used allowing the observation of their internal structure, and which microscope is combined with a light source that emits a beam of adjustable light, so that by means of such slit lamps different eye diseases can be detected.

Also, a tonometer useful to measure ocular pressure by applanation tonometry technique, a green diode laser to perform argon laser treatments in case of small retinal detachments, a laser for carrying out photodynamic therapies in cases of macular venous thrombosis and other type of attachments may be associated to the slit lamp in order to complement it.

Moreover, it can be stated that different types of toric axis markers of intraocular lenses on cornea prior to the implantation of an intraocular lens are known in the market, according to the prescription of an ophthalmologist after eye examination in order to determine its correct positioning, that is, the inclination (degrees) of the toric axis of the lens with respect to a horizontal plane, by trying to avoid the deviation of the axis of the toric intraocular lens with respect to the more curved meridian.

Once the corresponding intraocular lens is implanted by means of the thin beam of light emitted by the slit lamp, according to the inclination (degrees) determined with respect to a horizontal plane, the correct alignment of the lens can be checked by means of the graded scale that said slit lamps have.

Thus, among the different means used for marking the toric axis of an intraocular lens prior to its implantation in the eye, specific mention can be made of the one known as "pendulum", which is based on an element from which a pendulum hangs and at which the patient stares, wherein an "U"-shaped ink marking is made by means of a tool, which marking is always limited to the horizontal position of 0°-180° thereof.

A preoperative toric axis marker, which is based on a handle provided with a bubble level to determine a perfect subjective horizontal alignment of the marker but not of the patient and where the slit lamp is not necessary, can be also considered.

Similarly, we can consider that preoperative toric axis marker which is based on a semi-circular body having a front handle that is straight or inclined with respect to the body, which semi-circular body has three patterns of marks, namely, a central one and one at each side.

Another marker of the toric axis on the cornea is based on a circular crown-shaped first body, on which the degrees have been indicated and into which a second body is fitted for alignment and marking of the curviest axis and that inexorably requires a prior marking of 0°-180°.

Finally, reference can be made to that toric axis marker that is associated to the tonometer of a slit lamp, which is defined by a small cylindrical hollow body that is coupled to the tonometer at one of its bases, and has a pair of projections projecting by 180°, through which the marking is accomplished on the cornea by puncture, at its other free base.

Moreover, said cylindrical body presents, on its external surface, a gradation to determine the position in which the marking of the toric axis of the lens must be performed for its proper implantation, with the disadvantage that the gradual measure being from 10 ° to 10 °, because of its small diameter, does not allow for an adequate marking precision. This may cause the improper implantation of the intra-ocular lens and subsequent problems that are difficult to solve.

Furthermore, after every use thereof it must be sterilized, since it is not disposable.

### SUMMARY OF THE INVENTION

A preoperative toric axis corneal marker that is useful for determining the correct position of an intraocular lens, implanting an intrastromal ring in a patient's eye, performing a relaxing limbal incision or any other type of intervention requiring a precise marking prior to an intervention on the cornea is herein described, wherein the corneal marker is anchored by an attaching means to a slit lamp and which corneal marker comprises:
➢ a generally "L"-shaped body that:
   ✔ is anchored by a first wing to the attaching means to the slit lamp, and;
   ✔ a second wing has a hollow cylindrical shape, and;
➢ a tubular bushing is fitted into the second hollow cylindrical wing of the generally "L"-shaped body, which bushing presents, at its free base, at least one small lug in radial position.

In a first embodiment, the tubular bushing is simply fitted into the second hollow cylindrical wing of the generally "L"-shaped body, until it reaches an inner abutment.

In a second embodiment, the tubular bushing abuts onto a pressure element, such as, for example, a spring, in the second hollow cylindrical wing of the generally "L"-shaped body.

In a preferred embodiment, the tubular bushing will have, at its free base, a pair of small lugs diametrically positioned with an outer concave-curved surface for contacting the eye.

In another embodiment, an absorbent element, such as a thread or rubber filament, is attached to the outer concave-curved surface for contacting the eye of the pair of lugs, so that said absorbent element will be soaked with the eye marking ink in order to allow for making a clean mark.

In this way, the corneal marker will be associated to the slit lamp so that, by means of the slit lamp, the thin light beam will be projected on the eye through the inside of the second wing and the tubular bushing fitted into it, on the basis of the precise graduation predetermined by arranging the pair of diametrical lugs of the tubular bushing on the projected beam, which defines the toric axis of the lens to be implanted, and as it impacts on the eye, the beam will leave the marks on it according to a perfect graduation, what is a major advantage. This is because the grading scale of the slit lamp is very precise.

Also, the corneal marker is useful for implanting an intrastromal ring in a patient's eye, for performing a relaxing limbal incision or any other type of intervention that requires a precise marking prior to a surgery on the cornea.

In order to complement the description that is going to be carried out and with the purpose of facilitating the understanding of the characteristics of the invention, the present specification is accompanied by a set of drawings wherein their figures represent, by way of a non-limiting example, the most characteristic features of the invention:

### BRIEF DESCRIPTION OF THE DRAWINGS

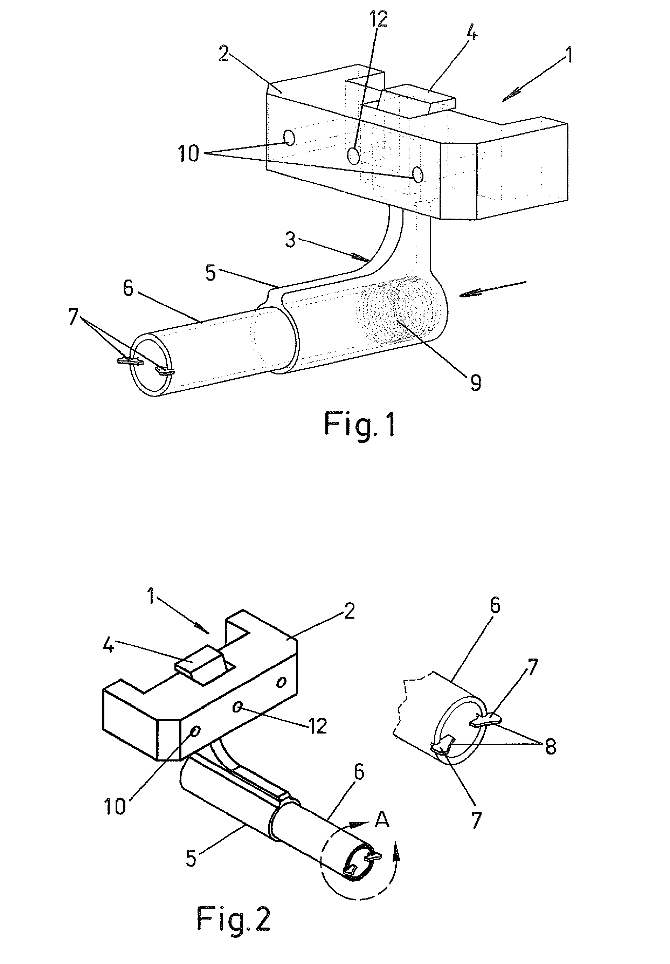

Figure 1 shows a first perspective view of a preoperative toric axis corneal marker according to the invention, which is anchored to an attaching means to the slit lamp, where the corneal marker, which is defined by a generally "L"-shaped body and a tubular bushing fitted into a second hollow cylindrical wing of said generally "L"-shaped body can be seen, and which tubular bushing may be biased by a spring.
Figure 2 shows a second perspective view of a preoperative toric axis corneal marker according to the invention, which is anchored to an attaching means to the slit lamp, where the tubular bushing, which presents a pair of small lugs in diametrical position and with a curved concave outer surface, can be seen in detail.

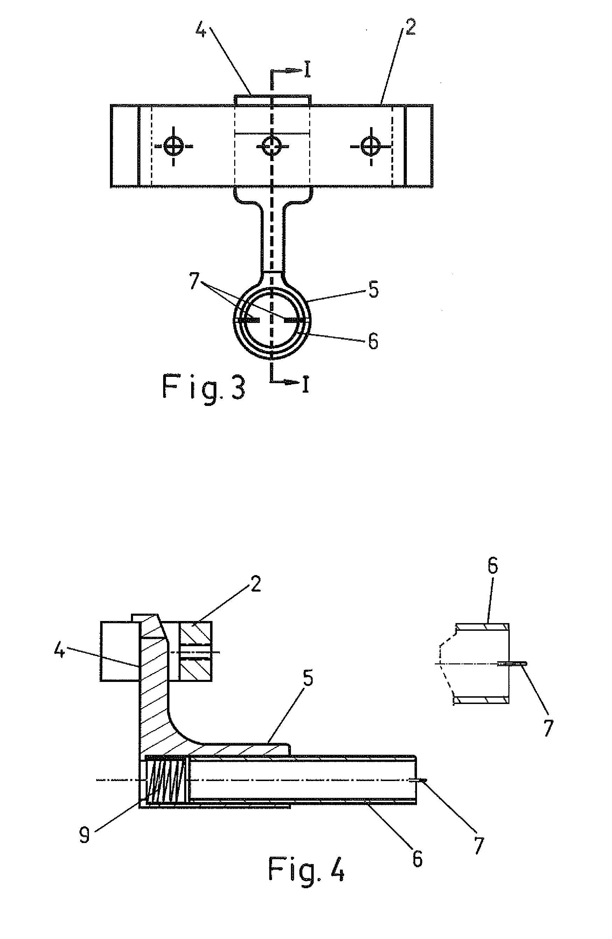

Figure 3 shows a front view of a preoperative toric axis corneal marker according to the invention.
Figure 4 shows a sectional view along the central vertical axis I-I of the previous figure, where the tubular bushing fitted into a second hollow cylindrical wing of the generally "L"-shaped body can be seen and which tubular bushing is put into stress by a spring of light pressure and where a radial lug of the pair of radial lugs arranged at the free base of the bushing is represented in detail.

### DESCRIPTION OF A PREFERRED EMBODIMENT

In view of the figures and in accordance with the numbering adopted, it can be seen the corneal marker 1 object of the invention, which is applicable for marking the toric axis of intraocular lenses on the eye in a preoperative phase, in order to allow for correctly implant of the appropriate intraocular lens to be implant thereof, so that the corneal marker 1 is anchored to an attaching means 2 to the slit lamp and which corneal marker 1 is basically formed by a generally "L"-shaped body 3 and a tubular bushing 6.

Thus, the generally "L"-shaped body 3 is anchored, by means of a first wing 4, to the attaching means 2 to the slit lamp and the second wing 5 has a hollow cylindrical shape, wherein a tubular bushing 6 is fitted and which bushing presents, at its outer free base, at least a small lug radially positioned, although in a preferred embodiment it will have a pair of lugs 7 diametrically positioned.

In this way, in a first embodiment, the tubular bushing 6 is fitted in the second hollow cylindrical wing 5 of the generally "L"-shaped body 3, its course being stopped by an inner fixed stop.

In a second embodiment the tubular bushing 6 is fitted in the second hollow cylindrical wing 5 of the generally "L"-shaped body 3 in abutment onto a pressing member 9, such as, for example, a spring, so that in use, when contacting the eye, it will have a short course in order to protect the integrity of the eye.

Moreover, the pair of lugs 7 diametrically arranged at the outer free base of the tubular bushing 6 has an outer curved-concave surface for contacting the eye, in order to adapt to the anatomy thereof.

In an embodiment, an absorbent element, such as a thread or rubber filament 8, can be fixed to the outer concave-curved surface for contacting the eye of the pair of lugs 7, so that said absorbent element will be soaked with the eye marking ink in order to allow for making a clean mark, which is indicative of the toric axis of positioning of the intraocular lens in the eye in order to achieve a perfect implantation. Also, as indicated, the corneal marker 1 is also applicable to other types of interventions.

In short, the corneal marker 1 is associated with the slit lamp so that a slim beam of light will be projected through the slit lamp on the eye according to a pre-set precise graduation for the correct implantation of the corresponding intraocular lens, by arranging the pair of diametrical lugs 7 on the beam projected towards the eye through the inside of the second hollow wing 5 of the generally "L"-shaped body 3 and the tubular bushing 6, for which it just suffice to look through the inside of the tubular bushing 6 and turn it so that the diameter defined by the pair of lugs 7 is on the light beam projected coinciding with the toric axis of the lens to be implanted, and, by impacting on the eye, it will leave marks according to a perfect graduation, what represents a major advantage. This is because the grading scale of the slit lamp, according to which the thin beam of light is projected, is very precise.

Furthermore, the tubular bushing 6, through which the marking is made, has a minimum cost, since it is disposable, so it is not necessary to carry out any sterilization after each use.

With reference to the attached figures, as can be seen from Figure 1 of the drawings, the tubular bushing 6, which is fitted in the second hollow cylindrical wing 5 of the generally "L"-shaped body 3 and internally abuts onto a spring 9, so that by rotating the bush 6 the pair of lugs 7 will be arranged according to the desired angle with respect to a longitudinal central plane.

It can also be seen that the attaching means 2 to the corresponding slit lamp presents holes 10 for carrying out its fastening by respective screws, whereas the attachment of the generally "L"-shaped body 3 thereto will be made by threaded bolt into a third hole 12. Logically, the attaching means 2 may have different configurations, since its only function is to serve as a means for anchoring the corneal marker 1 to the conventional slit lamp in the right place.

In Figure 2 of the drawings, it can also be seen that the bushing 6 fitted in the second hollow cylindrical wing 5 of the generally "L"-shaped body 3 may be rotated, as indicated by arrow "A", to arrange the pair of lugs 7 according to the suitable graduation plane for marking the toric axis of the intraocular lens to be implanted in a precise way, as the slit lamp works as above-stated.

## Claims

1. **Preoperative toric axis corneal marker** useful for determining the correct position of an intraocular lens, implanting an intrastromal ring in a patient's eye, performing a relaxing limbal incision or any other type of intervention requiring a precise marking prior to a cornea surgery, **characterized in that** the corneal marker (1) comprises an attaching means (2) to a slit lamp and which corneal marker comprises:
➢ a generally "L"-shaped body (3) that:
✔ is anchored by a first wing (4) to the attaching means (2) to the slit lamp, and;
✔ a second wing (5) that has a hollow cylindrical shape, and;
➢ a tubular bushing (6) that is fitted into the second hollow cylindrical wing (6) of the generally "L"-shaped body (3), which tubular bushing (6) presents, at its free base, at least one small lug (7) that is radially positioned.

2. **Preoperative toric axis corneal marker,** according to claim 1, **wherein** the tubular bushing (6) abuts onto a pressure means (9).

3. **Preoperative toric axis corneal marker,** according to claim 2, **wherein** the pressure means on the tubular bushing (6) is defined by a spring (9) of low pressure.

4. **Preoperative toric axis corneal marker,** according to claim 1, **wherein** the tubular bushing (6) presents a pair of lugs (7) that are diametrically positioned at its outer free base.

5. **Preoperative toric axis corneal marker,** according to claim 1 and claim 4, **wherein** the pair of lugs (7) present an outer concave-curved surface for contacting the eye.

6. **Preoperative toric axis corneal marker,** according to claim 1 and claim 5, **wherein** an absorbent element (8) is attached to the outer concave-curved surface for contacting the eye of the pair of lugs (7).
